# FASCICULE DE BREVET EUROPEEN

(11) **EP 3 016 707 B1**
(45) Date de publication et mention de la délivrance du brevet: **06.09.2017**
(21) Numéro de dépôt: 14750555.6
(22) Date de dépôt: 04.07.2014
(51) Int. Cl.: A61M 11/02, A61M 15/00, B05B 11/06

(54) **DISPOSITIF DE DISTRIBUTION DE PRODUIT FLUIDE OU PULVERULENT**
SPENDERVORRICHTUNG FÜR FLÜSSIGE ODER PULVERFÖRMIGE PRODUKTE
FLUID OR POWDERY PRODUCT DISPENSING DEVICE

(30) Priorité: 05.07.2013 FR 1356667
(43) Date de publication de la demande: 11.05.2016
(73) Titulaire: APTAR France SAS, 27110 Le Neubourg (FR)
(72) Inventeur: BAILLET, Matthieu, 76000 Rouen (FR); LE MANER, François, F-27400 La Vallee Montaure (FR); POULLAIN, Franck, F-27400 La Haye Malherbe (FR); KEHRER, Holger, 78333 Stockach (DE)
(74) Mandataire: CAPRI
(86) Numéro de dépôt international: PCT/FR2014/051738
(87) Numéro de publication internationale: WO 2015/001281

(56) Documents cités:
- EP-A1- 0 575 239
- WO-A1-02/45866
- WO-A1-91/12895
- WO-A1-99/46055
- FR-A1- 2 775 963
- FR-A1- 2 780 388
- FR-A1- 2 956 649
- FR-A6- 2 186 853
- US-A- 4 017 007

## Description

La présente invention concerne un dispositif de distribution de produit fluide ou pulvérulent, et plus particulièrement un dispositif pour distribuer unitairement une dose de produit contenu dans un réservoir à l'aide d'un écoulement d'air sous pression.

Le document WO 99/46055 divulgue un tel dispositif dans lequel un élément de fermeture sphérique, qui obture la sortie du réservoir, est expulsé par l'écoulement d'air créé par une chasse d'air. Pour l'utilisation d'un dispositif de distribution de poudre plus particulièrement, la pression d'air nécessaire pour actionner le dispositif devra être suffisamment élevée pour garantir la distribution complète de la dose, ainsi que son fractionnement si cela est nécessaire. Dans le dispositif susmentionné, la pression d'air nécessaire pour actionner le dispositif est déterminée par la résistance donnée par la bille pour être expulsée. Cette résistance est relativement difficile à contrôler et à prédéterminer puisqu'elle est dépendante du frottement entre la bille et son siège cylindrique dans lequel elle est emmanchée pour obturer de manière étanche ledit réservoir. Il peut par conséquent être nécessaire de minimiser l'interférence entre la sphère et son siège cylindrique, ce qui peut évidemment altérer l'efficacité de l'obturation. De plus, il peut être nécessaire de minimiser la profondeur et le positionnement de la sphère dans son siège afin de faciliter son expulsion. Il peut également être nécessaire de fournir une pression d'air relativement élevée qui n'est pas toujours facile à réaliser à l'aide d'un système de pompe ou d'un système de soufflet, notamment lorsque ces chasses d'air sont actionnées manuellement par le patient. De plus, la distribution, c'est à dire l'expulsion de la bille de son siège, peut se produire à des longueurs différentes de la course de la pompe ou du soufflet de la chasse d'air, de sorte que l'instant précis de la distribution du produit ne peut pas être toujours prédéterminé de manière exacte. Enfin, il y a une limitation dans le choix des matériaux pour la sphère et pour son siège.

Le document WO 02/45866 décrit un dispositif dans lequel une bille de fermeture est expulsée mécaniquement par une tige solidaire d'une chasse d'air. Cette mise en oeuvre présente plusieurs inconvénients. Ainsi, une chasse d'air étanche en toute position peut présenter un risque, notamment lors du transport ou du stockage, avec en cas de différence de pression entre l'intérieur et l'extérieur de la chasse d'air, un risque de déclenchement non souhaité de ladite chasse d'air. De plus, le dispositif du document WO 02/45866 n'est pas réutilisable, et l'ensemble du dispositif doit être jeté après son utilisation. Notamment pour des raisons écologiques et économiques, il peut être souhaitable d'avoir un dispositif réutilisable dans lequel le réservoir serait changé après chaque actionnement mais pas la chasse d'air.

Le document FR2186853 décrit un autre dispositif de l'art antérieur.

La présente invention a pour but de fournir un dispositif de distribution de produit fluide ou pulvérulent qui ne reproduit pas les inconvénients susmentionnés.

La présente invention a donc pour but de fournir un dispositif de distribution de produit fluide ou pulvérulent, qui soit simple et peu coûteux à fabriquer et à assembler.

La présente invention a aussi pour but de fournir un tel dispositif de distribution de produit fluide ou pulvérulent qui permette l'utilisation d'une chasse d'air très simple, peu coûteuse et fiable, et ne nécessitant pas la création d'un écoulement d'air particulièrement puissant.

La présente invention a encore pour but de fournir un tel dispositif de distribution de produit fluide ou pulvérulent qui puisse être utilisé plusieurs fois avec plusieurs réservoirs différents.

La présente invention a aussi pour but de fournir un dispositif de distribution de produit fluide ou pulvérulent, dans lequel l'élément de fermeture assure en toute occasion une fermeture étanche du réservoir au repos.

La présente invention a donc pour objet un dispositif de distribution de produit fluide ou pulvérulent tel que défini dans la revendication 1. Avantageusement, le réservoir est monté de manière amovible sur la chasse d'air, de sorte qu'après l'actionnement du dispositif, le réservoir vide peut être retiré de ladite chasse d'air et remplacé par un nouveau réservoir plein, ladite chasse d'air étant adaptée à revenir en position de repos pour permettre un nouvel actionnement avec ledit nouveau réservoir plein.

Ces caractéristiques et avantages et d'autres apparaîtront plus clairement au cours de la description détaillée suivante d'un mode de réalisation avantageux, faite en référence aux dessins joints, donnés à titre d'exemples non limitatifs, et sur lesquels :
- la figure 1 est une vue schématique en section transversale d'un exemple d'un dispositif de distribution de produit fluide ou pulvérulent non conforme à la présente invention, en position de repos,
- les figures 2 et 5 à 8 sont des vues similaires à celle de la figure 1, montrant différentes phases de l'actionnement,
- la figure 3 est une vue de détail en perspective de la chambre d'air du dispositif de la figure 1,
- la figure 4 est une vue similaire à celle de la figure 3, en section transversale,
- la figure 9 est une vue similaire à celle de la figure 8, montrant le retrait du réservoir vide,
- la figure 10 est une vue similaire à celle de la figure 9, montrant uniquement la chasse d'air après retrait du réservoir vide et un nouveau réservoir plein, avant assemblage,
- les figures 11 et 12 sont des vues similaires à celle de la figure 10, montrant l'insertion d'un nouveau réservoir plein,
- la figure 13 est une vue schématique en perspective de la pièce sécable du réservoir,
- la figure 14 est une vue similaire à celle de la figure 13, en section transversale,
- la figure 15 est une vue schématique découpée en perspective montrant un autre exemple d'un dispositif de distribution de produit fluide ou pulvérulent non conforme à la présente invention, en position de repos,
- la figure 16 est une similaire à celle de la figure 15, montrant plus particulièrement la chasse d'air,
- la figure 17 est une vue de détail en perspective de la chambre d'air du dispositif des figures 15 et 16,
- la figure 18 est une vue similaire à celle de la figure 17, en section transversale,
- les figures 19 à 22 sont des vues schématiques en section transversale d'un dispositif de distribution de produit fluide ou pulvérulent selon un mode de réalisation couvert par l'invention, respectivement en position de repos, en cours d'actionnement, en position de distribution et en cours de retour de la chasse d'air vers la position de repos,
- la figure 23 est une vue schématique en perspective de la chasse d'air du dispositif des figures 19 à 22, en position de repos,
- les figures 24 à 27 sont des vues schématiques en section transversale d'un autre exemple d'un dispositif de distribution de produit fluide ou pulvérulent non conforme à la présente invention, respectivement en position de repos, en cours d'actionnement, en position de distribution et après retour de la chasse d'air vers la position de repos,
- les figures 28 à 31 sont des vues schématiques en section transversale d'un autre exemple d'un dispositif de distribution de produit fluide ou pulvérulent non conforme à la présente invention, respectivement en cours d'actionnement, en position de distribution, en cours retour de la chasse d'air vers la position de repos, et avant mise en place d'un nouveau réservoir plein,
- les figures 32 et 33 sont des vues schématiques en section transversale d'un autre exemple d'un dispositif de distribution de produit fluide ou pulvérulent non conforme à la présente invention, respectivement en position de distribution et après retour de la chasse d'air vers la position de repos, et

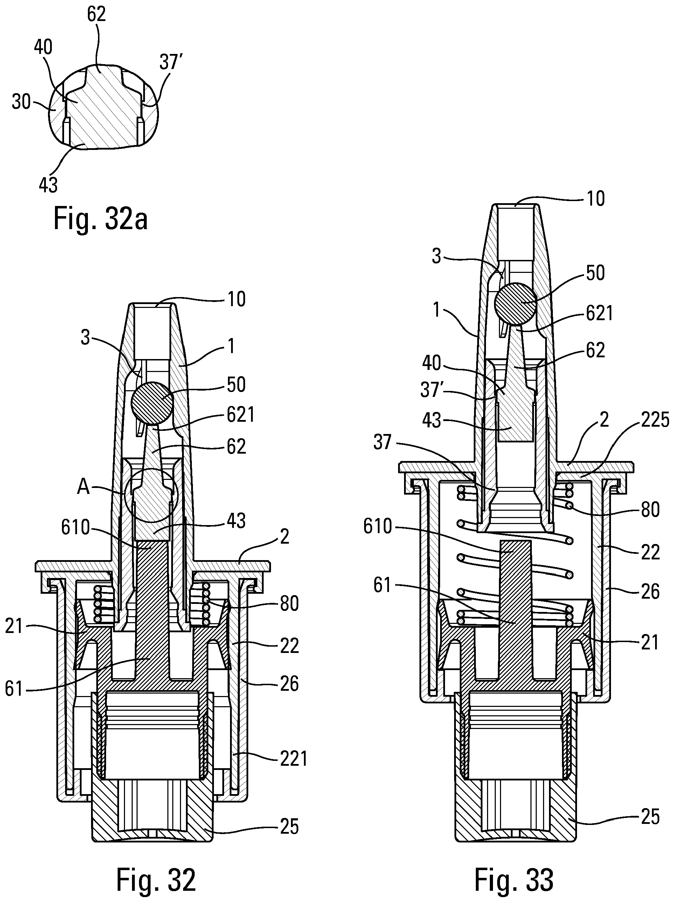

- la figure 32 a est une vue du détail A de la figure 32.

La présente invention, dans ses diverses variantes et modes de réalisation décrits ci-après, concerne plus particulièrement un dispositif du type de celui divulgué dans le document WO 02/45866. Ce document décrit le fonctionnement général du dispositif.

Il est toutefois entendu que la présente invention ne se limite pas à ce type de dispositif, mais est au contraire applicable à tous les types de dispositifs de distribution de produit fluide et pulvérulents comportant un réservoir obturé par un élément de fermeture, le contenu du réservoir devant être expulsé par un écoulement d'air.

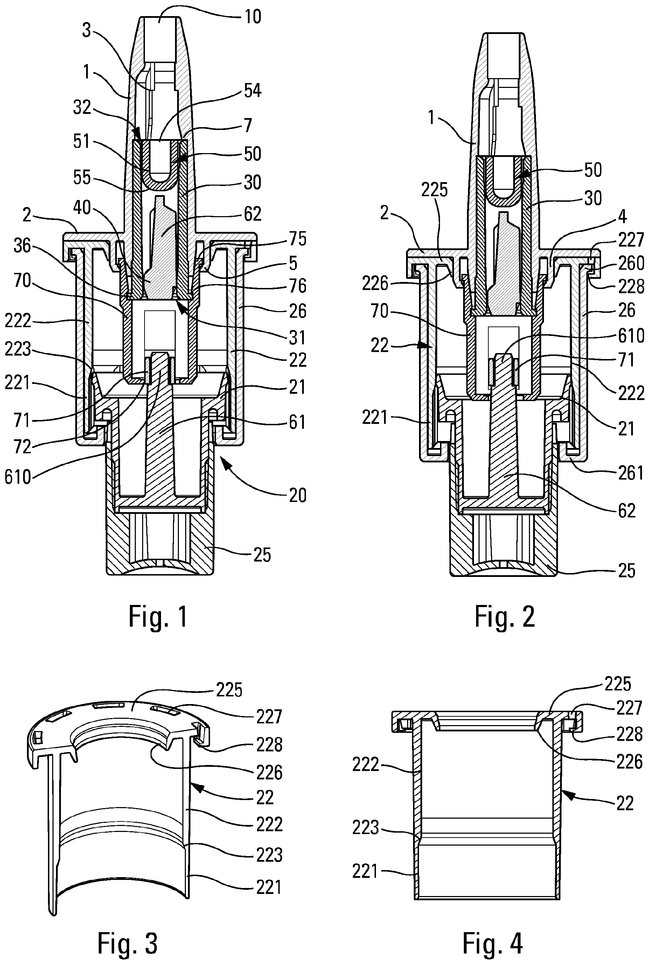

Il est entendu que dans toute la description, les termes « supérieur », « inférieur », « haut » et « bas » se réfèrent à la position droite du dispositif représenté notamment sur la figure 1.

En référence aux figures 1 à 14, il est représenté un exemple d'un dispositif de distribution de produit fluide ou pulvérulent non conforme à la présente invention. Le dispositif comporte un réservoir 30 comportant une entrée d'air 31 et une sortie de produit 32. L'entrée d'air 31 du réservoir est reliée à une chasse d'air 20 et la sortie de produit 32 du réservoir est reliée à une sortie de distribution 10 du dispositif. La sortie de produit 32 est obturée par un élément de fermeture 50 qui est emmanché à force dans ladite sortie de produit 32. L'entrée d'air 31 est pourvue d'un organe de retenue de produit 40 qui est adapté à maintenir le produit dans le réservoir avant l'actionnement du dispositif. La chasse d'air 20 est actionnée manuellement par l'utilisateur et est adaptée à créer un écoulement d'air qui va traverser le réservoir 30 pour emmener le produit qu'il contient en direction de la sortie de distribution 10.

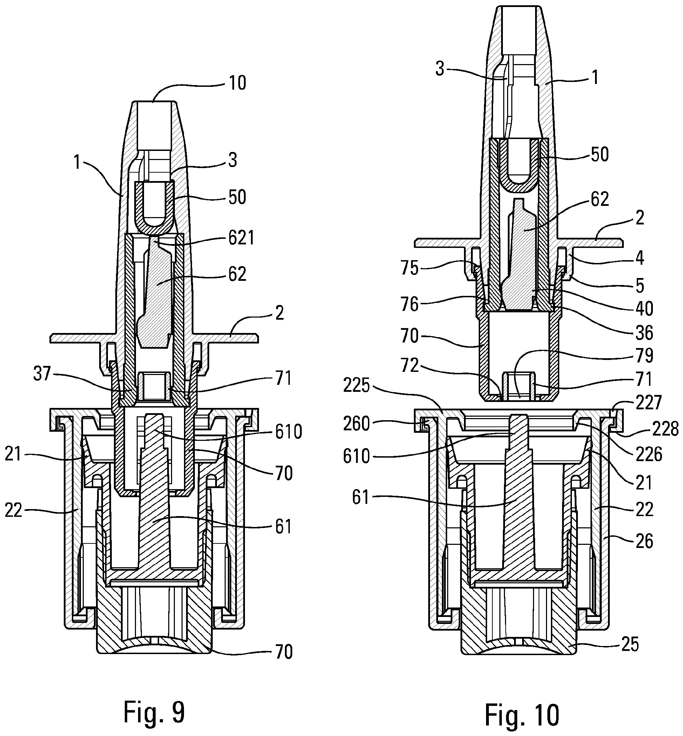

Le réservoir 30 est solidaire, notamment emmanché, dans une tête de distribution 1 qui comporte la sortie de distribution 10. Un épaulement radial 7 de la tête de distribution 1 définit avantageusement la position assemblée du réservoir 30 dans ladite tête de distribution 1. Une pièce sécable 70 est assemblée sur ledit réservoir 30 et sur ladite tête de distribution 1, formant ainsi un ensemble solidaire, comme visible sur la figure 10. Au niveau de l'entrée d'air 31, le réservoir comporte un bord radial saillant vers l'extérieur 36 adapté à se fixer à ladite pièce sécable 70, notamment par encliquetage dans une rainure 76 de la pièce sécable 70. Par ailleurs, ladite pièce sécable 70 comporte également un bord axial supérieur 75 qui se fixe à ladite tête de distribution, notamment par encliquetage sur un profil d'encliquetage 5 formé sur le bord inférieur d'une bride axiale 4 de la tête de distribution 1. La pièce sécable 70 comporte à son bord axial inférieur une ouverture axiale centrale 79 dans laquelle est fixé un élément témoin 71. Cet élément témoin 71 est relié audit bord axial inférieur par au moins un pont sécable 72. De préférence, il y a plusieurs ponts sécables 72, par exemple trois.

Le dispositif comporte un système d'ouverture mécanique 61, 62, qui est solidaire de la chasse d'air 20, c'est à dire qu'il est actionné simultanément à l'actionnement de ladite chasse d'air 20, et qui est adapté à coopérer avec ledit élément de fermeture 50 pour l'expulser mécaniquement de sa position d'obturation lors de l'actionnement du dispositif. Le système d'ouverture mécanique comporte un ensemble de tiges 61, 62, dont une première partie de tige 61 est solidaire de la chasse d'air 20, et une seconde partie de tige 62 est poussée par ladite première partie de tige 61 lorsque le dispositif est actionné. L'ensemble de tiges 61, 62 va en fin de course d'actionnement, c'est à dire en position de distribution, coopérer avec l'élément de fermeture 50 pour l'expulser mécaniquement de sa position d'obturation.

L'organe de retenue de produit 40 peut être avantageusement réalisé monobloc avec la seconde partie de tige 62. Ainsi, l'organe de retenue de produit 40 peut être réalisé de manière étanche au produit et étanche à l'air, avant l'actionnement du dispositif, la pression d'air créée par la chasse d'air 20 ne pénétrant à l'intérieur du réservoir 30 qu'au moment où ledit organe de retenu 40 est déplacé ensemble avec la seconde partie de tige 62, en étant poussé par la première partie de tige 61.

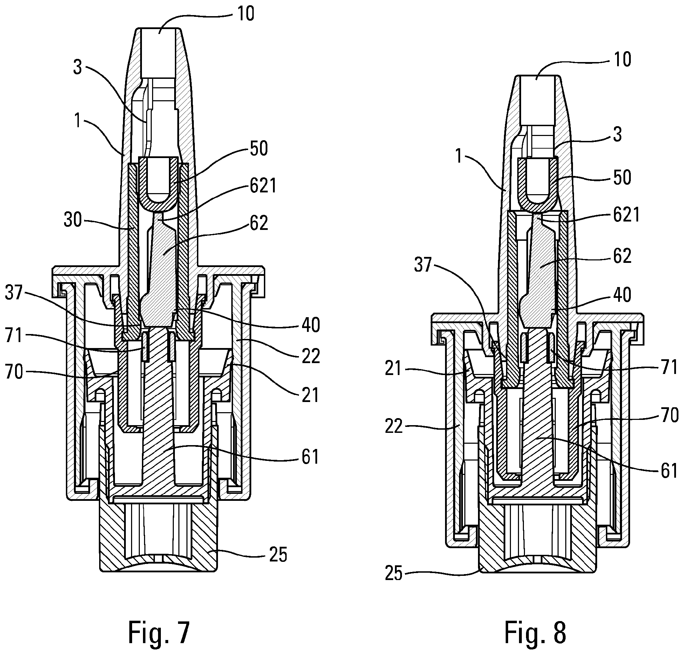

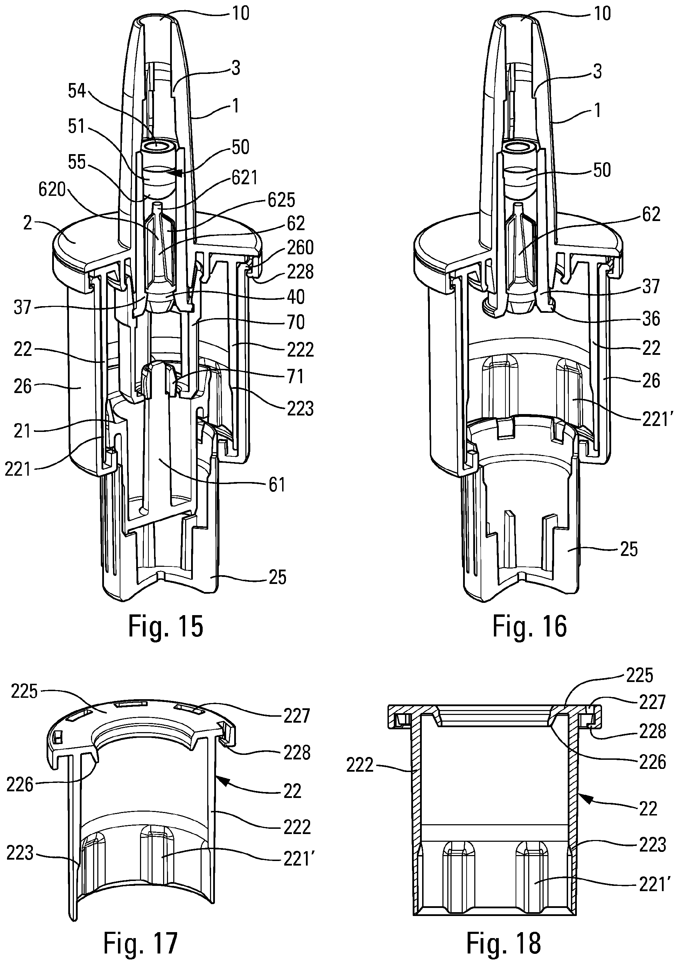

L'organe de retenue 40 est avantageusement réalisé sous la forme d'un disque rigide qui se prolonge à l'intérieur du réservoir par la seconde partie de tige 62. Cette seconde partie de tige 62 est de préférence réalisée avec une partie centrale 620, qui peut être conique ou d'une forme environ similaire, se terminant par une extrémité axiale supérieure 621, et plusieurs ailettes latérales 625, par exemple trois. Ces ailettes ne sont pas explicitement visibles sur les figures 1 à 14, mais elles sont visibles notamment sur la figure 15, qui montre en perspective la pièce formant l'organe de retenue 40 et la seconde partie de tige 62, et qui est tout à fait similaire à celle représentée en section transversale notamment sur la figure 1. Ces ailettes 625 assurent notamment un guidage de la seconde partie de tige 62 dans le corps du réservoir lors de l'actionnement. Avantageusement, ledit disque formant l'organe de retenue 40 est emmanché à force dans le réservoir 30. De préférence, comme visible notamment sur les figures 7 et 8, le réservoir 30 comporte à proximité de l'entrée d'air 31 un épaulement radial 37 définissant une partie inférieure de réservoir de diamètre interne réduit. L'organe de retenue 40 est emmanché à force dans cette partie inférieure de diamètre interne réduit en position de stockage et de repos, pour assurer une fermeture étanche du réservoir. Lorsque l'organe de retenue 40 est déplacé hors de sa position de repos, il ne coopère plus avec cette partie inférieure de diamètre interne réduit et ne ferme donc plus de manière étanche ladite entrée d'air 31 du réservoir. En variante, ledit organe de retenue pourrait être fixé, par exemple soudé, au corps du réservoir. La seconde partie de tige 62 comporte une extrémité axiale supérieure 621, qui va coopérer lors de l'actionnement avec l'élément de fermeture 50.

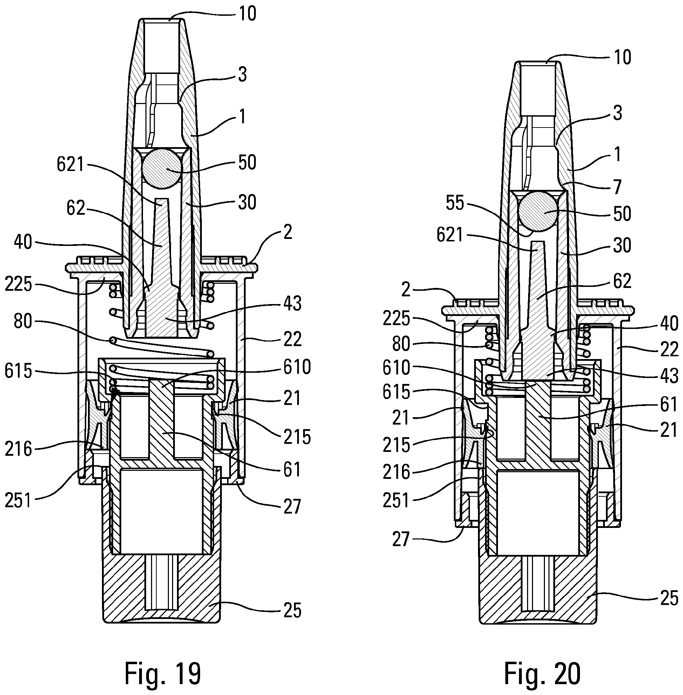

L'élément de fermeture 50 peut être sphérique, par exemple une bille plastique, comme représenté sur les figures 19 à 33, ou non sphérique, comme représenté sur les figures 1 à 16. Dans ce cas, l'élément de fermeture 50 peut comporter une partie cylindrique 51 qui définit une surface d'étanchéité périphérique avec ladite sortie de produit 32. L'étanchéité est donc formée non pas sur une ligne d'étanchéité, comme avec une bille sphérique, mais sur une surface cylindrique. L'exemple représenté sur les figures 1 à 16 montre un élément de fermeture 50 avantageux, comportant un manchon cylindrique axial creux. D'un coté axial, qui est celui tourné vers l'intérieur du réservoir 30 en position de fermeture, l'élément de fermeture 50 comporte une partie arrondie 55, notamment semi-sphérique, pour faciliter le passage du produit vers la sortie de distribution 10 lors de l'actionnement. De l'autre coté axial, le manchon creux est avantageusement ouvert 54. Cette mise en oeuvre donne une certaine souplesse à l'élément de fermeture 50, ce qui facilite sa mise en place et permet en partie de compenser les tolérances dimensionnelles de fabrication pour garantir une fermeture étanche en position de fermeture.

La chasse d'air représentée sur la figure 1 comporte un piston 21 coulissant dans une chambre d'air 22, le piston 21 étant actionné manuellement par l'utilisateur. Avantageusement, cet actionnement est réalisé au moyen d'un élément poussoir 25 assemblé sur ledit piston 21.

Le piston 21 est solidaire de la première partie de tige 61, avantageusement en étant formé sur une même pièce monobloc.

La chambre d'air 22, plus particulièrement visible sur les figures 3 et 4, comporte un corps cylindrique 222 comportant à son bord axial supérieur une bride radiale 225. Cette bride radiale 225 s'étend d'une part radialement vers l'intérieur dudit corps cylindrique 222, où elle comporte une extension axiale 226 s'étendant axialement vers le bas. Cette extension axiale 226 a pour but de coopérer, avantageusement de manière sensiblement étanche, avec la surface externe de la bride axiale 4 de la tête de distribution 1, lorsque le dispositif est assemblé. La bride radiale 225 s'étend d'autre part radialement vers l'extérieur dudit corps cylindrique 222, où elle comporte des moyens de fixation 228 à un corps externe 26 assemblé autour de ladite chambre d'air 22. Avantageusement, ces moyens de fixation comportent un ou plusieurs profil(s) d'encliquetage 228 adapté(s) à s'encliqueter sur un bord radial supérieur 260 dudit corps externe 26. Avantageusement, pour simplifier la fabrication, plusieurs profils d'encliquetage 228 sont répartis autour de la périphérie de ladite bride radiale 225 en étant face à des fenêtres correspondantes 227 qui simplifient le moulage de la pièce formant chambre d'air, en évitant notamment d'avoir à utiliser des moules à tiroirs complexes.

En position de repos, visible sur la figure 1, la chasse d'air 20 est ouverte sur l'atmosphère. Dans l'exemple de la figure 1, cette ouverture sur l'atmosphère est obtenue par une partie cylindrique de plus grand diamètre 221 du corps cylindrique de la chambre d'air 22. Dans cette partie de plus grand diamètre 221, prévue au niveau du bord axial inférieur de la chambre d'air 22, le piston 21 de la chasse d'air 20 ne coopère pas de manière étanche avec ladite chambre d'air. Lorsque le dispositif est actionné, le piston 21 va se déplacer hors de ladite partie de plus grand diamètre 221 et ainsi coopérer de manière étanche avec une partie de corps cylindrique 222 de la chambre d'air 22, pour ainsi comprimer l'air contenu dans la chambre d'air. Avantageusement, la transition entre la partie de corps cylindrique 222 et la partie de plus grand diamètre 221 est réalisée de manière progressive, par exemple par une partie de paroi conique 223, afin de faciliter le déplacement du piston hors de sa position de repos. Le corps externe 26 comporte avantageusement à son bord axial inférieur un rebord radial 261 tourné vers l'intérieur, qui permet notamment de protéger la chasse d'air 20 en position de repos, en particulier la partie de plus grand diamètre 221 de la chambre d'air 22 et le piston 21.

Lorsque le dispositif est assemblé, comme notamment visible sur la figure 1, ledit élément témoin 71 de la pièce sécable 70 est emmanché autour d'une extrémité rétrécie 610 de la première partie de tige 61. Les ponts sécables 72 sont adaptés à se casser lors de l'actionnement du dispositif, comme visible sur les figures 1 et 2.

L'actionnement du dispositif est illustré sur les figures 2 et 5 à 8. Ainsi, lorsque l'utilisateur souhaite actionner le dispositif, il place d'une part ses doigts sur le repose-doigt 2 de la tête de distribution 1 et d'autre part son pouce sur l'élément poussoir 25, et il exerce une force d'actionnement, qui va d'abord casser les ponts sécables 72 puis déplacer la première partie de tige 61 et le piston 21 vers la position de distribution. Dés le début de l'actionnement, visible sur la figure 2, le piston 21 de la chasse d'air va coopérer de manière étanche avec la chambre d'air 22, de sorte que l'air contenu dans ladite chambre d'air 22 va être progressivement comprimé au cours de l'actionnement.

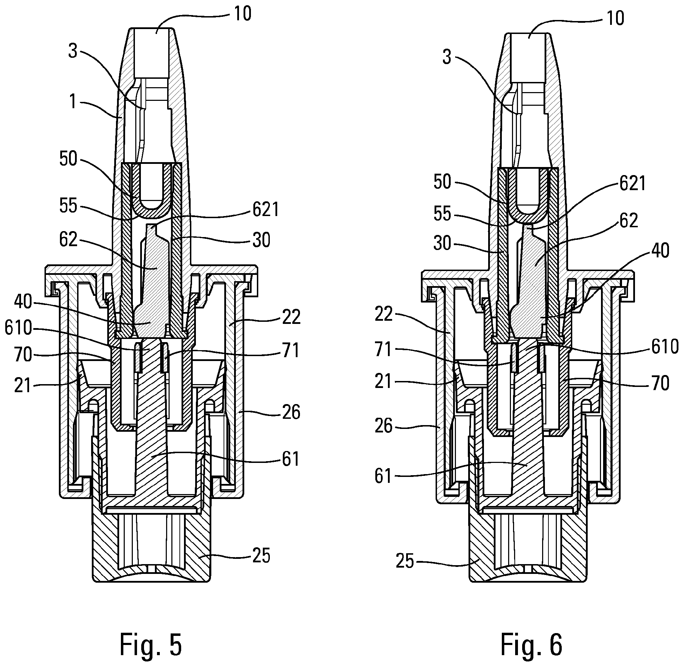

Dans la position de la figure 5, l'extrémité axiale supérieure 610 de la première partie de tige vient en contact de l'organe de retenue 40 et donc de la seconde partie de tige 62.

Une poursuite de l'actionnement déplace ledit organe de retenue 40 axialement vers le haut dans le réservoir 30, donc hors de sa position d'obturation ou de fermeture étanche de l'entrée d'air, comme visible sur la figure 6. A ce moment là, l'air comprimé dans la chambre d'air 22 peut donc pénétrer dans le réservoir 30. Au même moment, l'extrémité axiale supérieure 621 de la seconde partie de tige 62 vient en contact de la partie arrondie 55 de l'élément de fermeture 50.

Une poursuite de l'actionnement va donc déplacer l'élément de fermeture 50 axialement vers le haut, hors de sa position d'obturation, comme visible sur la figure 7.

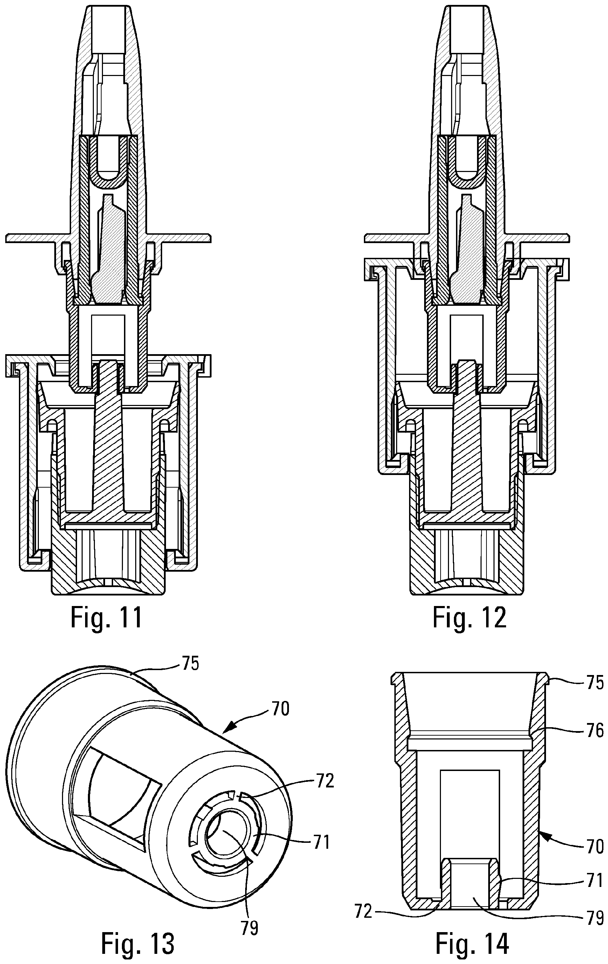

Lorsque l'étanchéité de l'élément de fermeture 50 est rompue, celui-ci est expulsé hors du réservoir 30 pour permettre la distribution du produit fluide ou pulvérulent sous l'effet de l'air comprimé. L'élément de fermeture 50 vient alors se coincer dans des nervures 3 de la tête de distribution 1, qui empêchent notamment tout risque d'expulsion dudit élément de fermeture hors de ladite tête de distribution 1. La position de distribution, en fin de course d'actionnement et après distribution du produit, est représentée sur la figure 8. L'exemple représenté sur les figures 1 à 14 illustre un dispositif rechargeable. Ainsi, en position de distribution représentée sur la figure 8, l'utilisateur peut retirer l'ensemble formé par la tête de distribution 1, le réservoir 30 et le corps sécable 70 de la chasse d'air. L'élément témoin 71 reste dans le réservoir 30, retenu notamment par ledit épaulement radial 37, et la pièce sécable 70 coulisse le long de la première partie de tige 61 pour s'en séparer. Après retrait du réservoir vide, illustré sur la figure 10, un nouveau réservoir plein peut être assemblé sur la chasse d'air. La figure 11 montre un ensemble formé par une tête de distribution 1, un réservoir 30 et une pièce sécable 70 dont l'élément témoin 71 est positionné autour de l'extrémité rétrécie 610 de la première partie de tige 61. L'utilisateur insère alors ce nouvel ensemble dans la chasse d'air, poussant ainsi la première partie de tige 61 vers le bas, ce qui provoque donc le coulissement du piston 21 en retour vers sa position de repos. Pour ce faire, la résistance au retour du piston 21 est inférieure à celle nécessaire pour casser les ponts sécables 72 de la pièce sécable 70. En fin d'insertion, juste avant d'arriver en position de repos de la figure 1, la bride axiale 4 de la tête de distribution 1 vient coopérer avec l'extension axiale 226 de la chambre d'air. La fin de l'insertion déplace ensuite le piston 21 vers la partie de plus grand diamètre 221 pour ainsi supprimer l'étanchéité à cet endroit, et la chasse d'air se retrouve donc ouverte sur l'atmosphère en position de repos, comme visible sur la figure 1. Le dispositif est alors prêt pour un nouveau cycle d'actionnement. Cet exemple est avantageux en ce qu'il ne comporte aucun ressort métallique. De plus, l'élément témoin 71 forme un témoin d'usage qui informe l'utilisateur que le réservoir a été vidé lorsqu'il est disposé dans ledit réservoir. Cet élément témoin 71 peut donc avantageusement être réalisé de manière à être aisément identifiable, par exemple dans une couleur particulière. Un autre avantage est donné par la présence de la pièce sécable, qui protège le réservoir et l'organe de retenue 40 avant utilisation, et notamment au moment de son insertion dans la chasse d'air.

En référence aux figures 15 à 18, il est représenté un autre exemple d'un dispositif de distribution de produit fluide ou pulvérulent non conforme à la présente invention. Cet exemple est très proche de l'exemple des figures 1 à 14, la seule différence étant la mise à l'air libre de la chasse d'air 20 en position de repos, qui ici n'est pas réalisée au moyen d'une partie cylindrique de plus grand diamètre du corps de la chambre d'air 22, mais par des rainures 221' ménagées dans la partie inférieure du corps cylindrique 222 de la chambre d'air 22. Ainsi, au lieu d'être périphérique comme dans l'exemple des figures 1 à 14, la mise en relation de la chambre d'air 22 avec l'atmosphère en position de repos est ici obtenue par des passages d'air fournis par lesdites rainures 221', réparties autour de la périphérie. Les figures 17 et 18 illustrent plus particulièrement ces rainures 221', avec lesquelles le piston 21 va coopérer en position de repos.

En référence aux figures 19 à 23, il est représenté un dispositif de distribution de produit fluide ou pulvérulent selon un mode de réalisation avantageux de la présente invention. Ce dispositif diffère de celui de l'exemple des figures 1 à 14 par plusieurs aspects. Ainsi, l'élément de fermeture 50 est sphérique, notamment une bille. La seconde partie de tige 62 ne comporte pas d'ailettes, et l'organe de retenue 40 se prolonge vers le bas par une extension axiale 43, qui vient en contact de l'extrémité axiale supérieure 610 de la première partie de tige 61 lors de l'actionnement.

Par ailleurs, il n'y a pas ici de corps externe, mais un simple cache 27 assemblé sur le bord axial inférieur de la chambre d'air 22.

Selon l'invention, le piston 21 est séparé de la première partie de tige 61, et coulisse à la fois par rapport à la chambre d'air 22 et à une surface cylindrique 614 solidaire de la première partie de tige 61. La chambre d'air 22 est parfaitement cylindrique, la mise à l'air libre en position de repos de la chambre d'air 22 étant réalisé au niveau de cannelures ou rainures 615 formées sur ladite surface cylindrique 614 et qui coopèrent avec le piston 21, notamment en position de repos. Le piston 21 comporte ainsi une lèvre interne 215 qui coulisse de manière étanche sur la surface cylindrique 614 lors de l'actionnement et qui coopère avec lesdites cannelures 615 en position de repos. Le piston 21 comporte aussi une extension axiale 216 qui coopère avec un bord supérieur 251 de l'élément poussoir 25, qui va déplacer ledit piston 21 dans la chambre d'air 22 lors de l'actionnement.

Un ressort 80 est prévu entre la bride radiale 225 de la chambre d'air 22 et la pièce formant première partie de tige 61 et surface cylindrique 614, pour ramener la chasse d'air automatiquement en position de repos après actionnement.

Le principe de fonctionnement est le suivant.

En position de repos de la figure 19, le réservoir 30 est fermé de manière étanche par l'organe de retenue 40 et par l'élément de fermeture 50. La chasse d'air est ouverte sur l'atmosphère de par la coopération entre la lèvre interne 215 du piston 21 et les cannelures 615 de la surface cylindrique 614.

Lorsque l'utilisateur actionne le dispositif il appuie sur l'élément poussoir 25, comme pour les modes de réalisation précédents. Pendant cette course initiale, la lèvre interne 215 du piston va quitter les cannelures 615 pour venir coopérer de manière étanche avec la surface cylindrique 614, fermant ainsi la chambre d'air 22. Au même moment, le bord supérieur 251 de l'élément poussoir 25 vient en contact de l'extension axiale 216 du piston 21, et l'extrémité axiale supérieure 610 de la première partie de tige 61 vient en contact de l'extension axiale 43 de l'organe de retenue 40. Par contre, l'extrémité axiale supérieure 621 de la seconde partie de tige 62 n'est pas encore en contact de la surface arrondie 55 de l'élément de fermeture 50. Ceci est visible sur la figure 20.

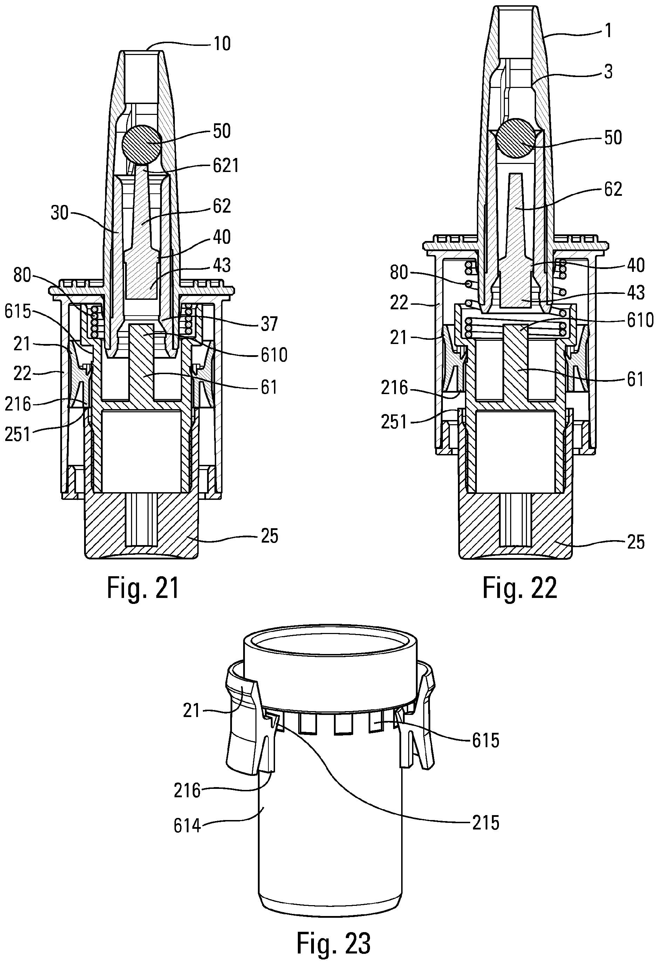

Une poursuite de l'actionnement va donc simultanément déplacer le piston 21 dans la chambre d'air pour ainsi comprimer l'air qu'elle contient, et déplacer l'organe de retenue 40 hors de sa position de fermeture du réservoir 30. Lorsque la seconde partie de tige 62 contacte l'élément de fermeture 50, celui-ci est expulsé mécaniquement de sa position d'obturation, pour permettre l'expulsion du produit sous l'effet de l'air comprimé par la chasse d'air. La position de distribution est représentée sur la figure 21. Comme visible sur cette figure 21, l'organe de retenue 40 peut se détacher de la première partie de tige 61 lors de l'expulsion du produit, sous l'effet de l'air comprimé fournit par la chasse d'air.

Lorsque l'utilisateur relâche le dispositif, le ressort 80 qui a été comprimé pendant l'actionnement va ramener la première partie de tige 61 vers sa position de repos. Ceci crée une dépression qui va aspirer l'élément de fermeture 50 et l'organe de retenue 40 en retour vers ou proche de leurs positions de fermeture. Ceci bloque ainsi le chemin de ré-aspiration pour éviter de souiller la chasse d'air lors du retour automatique en position de repos alors que le réservoir vide est toujours assemblé sur la chasse d'air. Le piston 21 par contre va rester dans sa position de distribution en raison des frottements avec la chambre d'air 22 et de la dépression créée dans le réservoir 30, de sorte que la surface cylindrique 614 va glisser sur la lèvre interne 215 du piston jusqu'à ce que celle-ci coopère à nouveau avec les cannelures 615. A ce moment là, la chambre d'air 22 est à nouveau à l'air libre et il n'y a plus de dépression créée par le retour en position de repos. Le piston 21 est alors entrainé également vers sa position de repos. Ceci permet de refermer le réservoir après utilisation.

Eventuellement, l'unité formée par la tête de distribution 1 et le réservoir 30 vide pourrait être retirée de la chasse d'air et remplacée par une nouvelle unité comportant un réservoir plein.

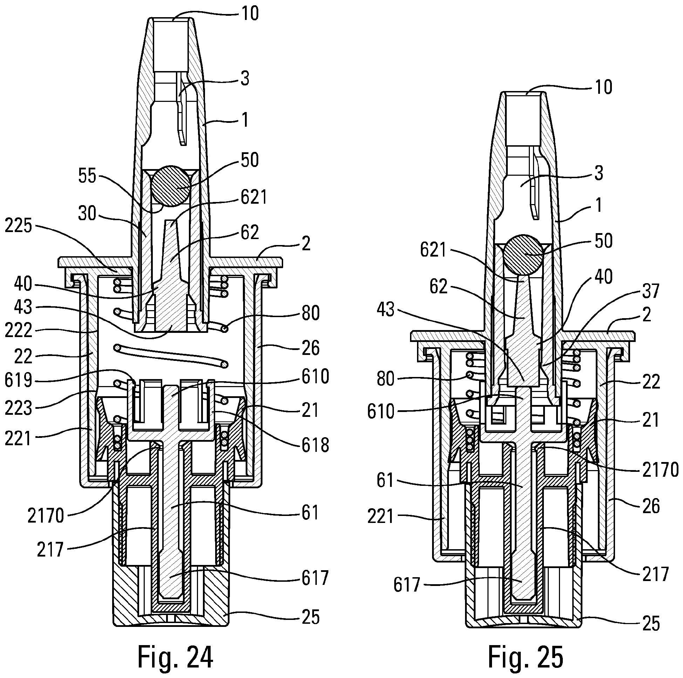

En référence aux figures 24 à 27, il est représenté un autre exemple d'un dispositif de distribution de produit fluide ou pulvérulent non conforme à la présente invention. Dans cet exemple, l'unité formée par la tête de distribution 1 et le réservoir 30 est similaire à celle décrite ci-dessus en référence au mode de réalisation avantageux de la présente invention représenté sur les figures 19 à 23. Le piston 21 est à nouveau séparé de la première partie de tige 61. Le piston 21 coulisse dans une chambre d'air 22 sensiblement similaire à celle de l'exemple des figures 1 à 14, à savoir avec une partie de plus grand diamètre au niveau de son bord axial inférieur, pour assurer la mise à l'air libre de la chambre d'air en position de repos. Ceci n'est toutefois qu'un exemple de réalisation.

Un ressort 80 est disposé entre la bride radiale 225 de la chambre d'air 22 et le piston 21, pour ramener le piston 21 automatiquement en position de repos après l'actionnement.

Le piston 21 est solidaire d'un manchon creux 217 pourvu à son extrémité axiale supérieure d'un épaulement de retenue 2170. La partie inférieure de la première partie de tige 61 est adaptée à coulisser dans ledit manchon creux 217. Elle comporte à son extrémité axiale inférieure une projection radiale 617 qui coopère avec ledit épaulement de retenue 2170 pour maintenir ladite projection radiale 617 de ladite première partie de tige 61 à l'intérieur dudit manchon creux 217. La partie supérieure de ladite première partie de tige comporte ladite extrémité axiale supérieure 610 qui coopère avec l'extension axiale 43 de l'organe de retenue 40 lors de l'actionnement. Cette partie supérieure comporte aussi plusieurs pattes axiales formant un manchon externe creux 618 entourant ladite extrémité axiale supérieure 610, et qui peuvent comporter éventuellement des moyens d'encliquetage 619.

En position de repos de la figure 24, la chambre d'air 22 est ouverte sur l'atmosphère et le réservoir 30 est fermé, comme précédemment.

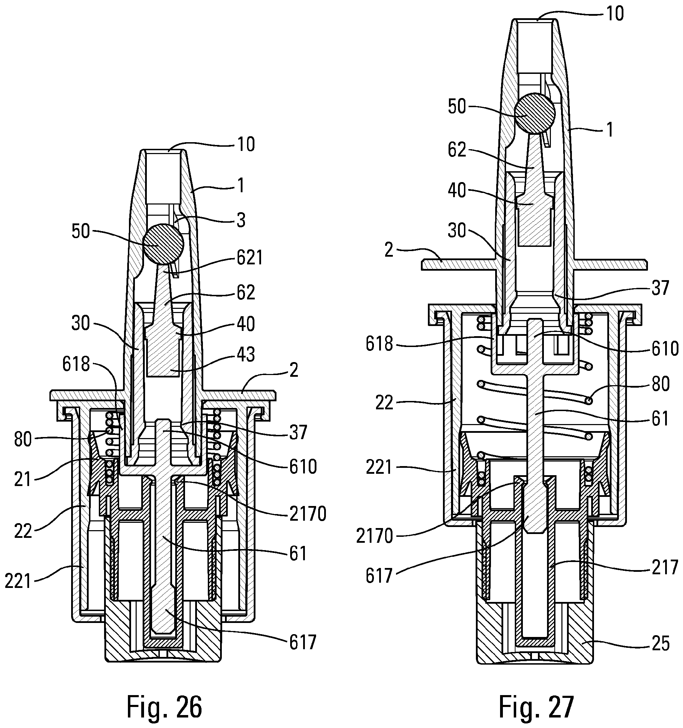

Lors de l'actionnement, la chambre d'air 22 va se fermer, permettant ainsi au piston 21 de la chasse d'air de comprimer l'air contenu dans la chambre d'air 22 ainsi que le ressort 80. Parallèlement, l'extrémité axiale supérieur 610 de la première partie de tige entre en contact avec l'organe de retenue 40 via son extension axiale 43. Le manchon externe 618 s'emmanche autour du bord inférieur du réservoir 30 et de la tête de distribution 1, comme visible sur les figures 25 et 26. La figure 26 montre la position de distribution, dans laquelle l'élément de fermeture 50 a été poussé par la seconde partie de tige 62 hors de sa position d'obturation, permettant au produit d'être expulsé à travers l'ouverture de distribution par l'écoulement d'air comprimé crée par la chasse d'air.

Lorsque l'utilisateur relâche le dispositif, le ressort 80 va ramener le piston 21 vers sa position de repos. Pendant ce mouvement, le manchon externe 618 de la première partie de patte 61 reste coincé sur l'unité formé du réservoir 30 et de la tête de distribution 1. La première partie de tige 61 n'est donc pas ramenée vers sa position de repos, et elle coulisse dans le manchon creux 217, comme visible sur la figure 27. Le manchon externe 618 bloque ainsi le chemin de ré-aspiration pour éviter de souiller la chasse d'air. Lorsque l'utilisateur retire le réservoir vide, il va déclipser ou décoincer ladite première partie de tige 61, qui peut alors revenir vers sa positon de repos par exemple par gravité. Etant donné que dans la position de repos du piston il n'y a plus de dépression puisque la chambre d'air 22 est reliée à l'atmosphère, il n'y a pas de risque de ré-aspiration lors du changement de réservoir.

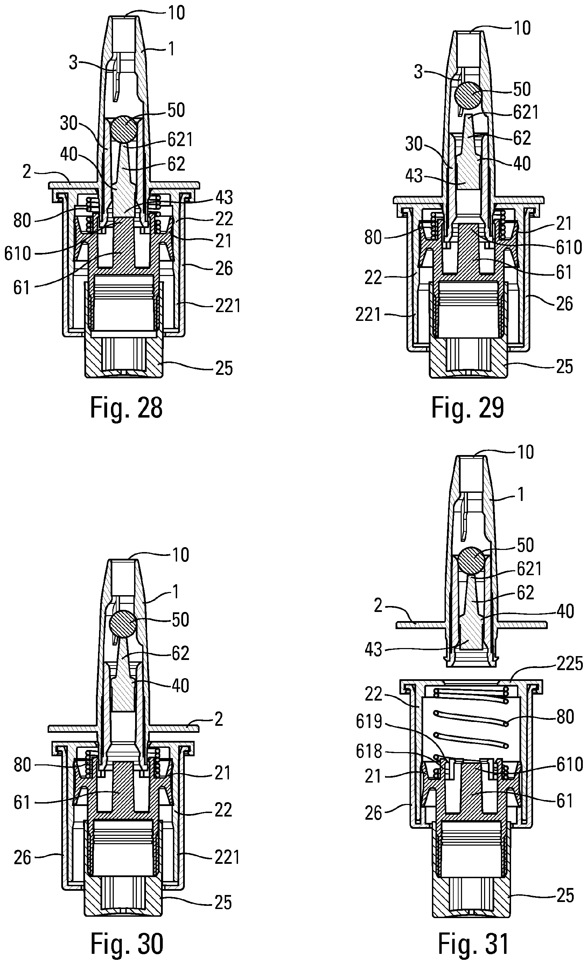

En référence aux figures 28 à 31, il est représenté un autre exemple d'un dispositif de distribution de produit fluide ou pulvérulent non conforme à la présente invention. Dans cet exemple, le piston 21 est à nouveau solidaire de la première partie de tige 61, et il comporte le même manchon externe 618 que celui décrit ci-dessus en référence aux figures 24 à 27. Lors de l'actionnement, c'est donc le piston 21 et la première partie de tige 61 qui s'emmanchent autour du réservoir et qui sont retenu dans cette position après distribution, lorsque l'utilisateur relâche son effort d'actionnement. Dans cette variante, le ressort 80 reste comprimé jusqu'à ce qu'on retire le réservoir vide. Des moyens d'encliquetage 619 sur lesdites pattes axiales du manchon externe 618 sont donc avantageux pour garantir une position stable malgré la présence du ressort comprimé. Ces moyens d'encliquetage 619 peuvent notamment coopérer avec un profil approprié sur le réservoir ou sur la tête de distribution. lorsque l'utilisateur retire le réservoir vide, comme visible sur la figure 31, le manchon externe 618 se désencliquette, et le ressort ramène la chasse d'air en position de repos.

En référence aux figures 32 à 33, il est représenté un autre exemple d'un dispositif de distribution de produit fluide ou pulvérulent non conforme à la présente invention. Dans cet exemple, la chasse d'air est similaire à celle du précédent exemple des figures 28 à 31, avec la première partie de tige 61 solidaire du piston 21. Il n'y a toutefois pas ici de manchon externe qui vient bloquer le piston et la première partie de tige sur le réservoir en position de distribution. Lorsque l'utilisateur a terminé l'actionnement, le ressort 80 ramène donc automatiquement la chasse d'air en position de repos comme visible sur la figure 33. Pour éviter une ré-aspiration lors de ce retour, le réservoir 30 comporte un second épaulement radial 37' décalé axialement vers le haut par rapport à l'épaulement axial 37 qui coince l'organe de retenue en position de fermeture avant actionnement. Ce second épaulement radial 37' est visible notamment sur la figure 32a. il permet de coincer l'organe de retenue 40 en position de distribution, ce qui évite une ré-aspiration dans la chasse d'air.

Bien que destiné principalement à la distribution d'un produit pulvérulent, la présente invention s'applique également à la distribution de produits liquides.

La présente invention a été décrite en référence à un mode de réalisation avantageux représenté sur les figures 19 à 23, mais il est entendu qu'un homme du métier peut y apporter toutes modifications, sans sortir du cadre de la présente invention tel que défini par les revendications annexées.

## Revendications

1. Dispositif de distribution de produit fluide ou pulvérulent comportant une sortie de distribution (10), une chasse d'air (20) pour générer un écoulement d'air lors de l'actionnement du dispositif, ladite chasse d'air (20) comportant un piston (21) coulissant dans une chambre d'air (22) entre une position de repos et une position de distribution, ladite chambre d'air (22) comportant un corps cylindrique (222) dans lequel ledit piston (21) coulisse de manière étanche, et au moins un réservoir (30) contenant une dose unique de produit, ledit réservoir (30) comportant une entrée d'air (31) reliée à ladite chasse d'air (20) et une sortie de produit (32) reliée à ladite sortie de distribution (10), ladite entrée d'air (31) comportant un organe de retenue de produit (40) pour maintenir le produit dans le réservoir (30) jusqu'à la distribution du produit, et ladite sortie de produit (32) étant obturée par un élément de fermeture (50) emmanché à force dans la sortie de produit (32) du réservoir (30), ledit dispositif comportant un système d'ouverture mécanique (61, 62) coopérant avec ledit élément de fermeture (50) pour l'expulser mécaniquement de sa position d'obturation lors de l'actionnement du dispositif, ledit système d'ouverture mécanique comportant un ensemble de tiges (61, 62) dont une première partie de tige (61) est solidaire de ladite chasse d'air (20) et coulissante dans ladite chambre d'air (22) lors de l'actionnement du dispositif, et une seconde partie de tige (62) est poussée par ladite première partie de tige (61) lors de l'actionnement du dispositif, l'ensemble de tiges (61, 62) coopérant en fin de course d'actionnement avec l'élément de fermeture (50) pour l'expulser mécaniquement de sa position d'obturation, ledit piston (21) de ladite chasse d'air (20), en position de repos, coopérant de manière non étanche avec ladite chambre d'air (22), de sorte que ladite chambre d'air (22) est reliée à l'atmosphère en position de repos, ledit piston (21) étant séparé de la première partie de tige (61) et coulissant à la fois par rapport à la chambre d'air (22) et à une surface cylindrique (614) solidaire de la première partie de tige (61), ledit piston (21) comportant une lèvre interne (215) qui coulisse de manière étanche sur la surface cylindrique (614) lors de l'actionnement du dispositif et qui coopère de manière non étanche avec des cannelures (615) formées sur ladite surface cylindrique (614) en position de repos pour mettre à l'air libre la chambre d'air (22) en position de repos, ledit piston (21) coopérant de manière étanche avec ledit corps cylindrique (222) en toutes positions, et coopérant de manière non étanche avec ladite surface cylindrique (614) uniquement en position de repos.

2. Dispositif selon la revendication 1, dans lequel ledit réservoir (30) est monté de manière amovible sur ladite chasse d'air (20), de sorte qu'après l'actionnement du dispositif, le réservoir vide peut être retiré de ladite chasse d'air et remplacé par un nouveau réservoir plein, ladite chasse d'air (20) étant adaptée à revenir en position de repos pour permettre un nouvel actionnement avec ledit nouveau réservoir plein.

## Patentansprüche

1. Ausgabevorrichtung für ein fluides oder pulverförmiges Produkt, aufweisend eine Ausgabeöffnung (10), einen Blasebalg (20) zum Erzeugen eines Luftstroms bei Betätigung der Vorrichtung, wobei der Blasebalg (20) einen Kolben (21) aufweist, der in einer Luftkammer (22) zwischen einer Ruheposition und einer Ausgabeposition gleitet, wobei die Luftkammer (22) einen zylindrischen Körper (222), in dem der Kolben (21) dichtend gleitet, und mindestens einen Behälter (30) aufweist, der eine einzelne Produktdosis enthält, wobei der Behälter (30) einen Lufteinlass (31), der mit dem Blasebalg (20) verbunden ist, und einen Produktauslass (32), der mit der Ausgabeöffnung (10) verbunden ist, aufweist, wobei der Lufteinlass (31) eine Produktrückhalteeinrichtung (40) aufweist, um das Produkt bis zur Ausgabe des Produkts in dem Behälter (30) zu halten, und der Produktauslass (32) durch ein Verschlusselement (50) versperrt ist, welches mit Kraft in den Produktauslass (32) des Behälters (30) eingedrückt ist, wobei die Vorrichtung ein mechanisches Öffnungssystem (61, 62) aufweist, das mit dem Verschlusselement (50) zusammenwirkt, um dieses bei Betätigung der Vorrichtung mechanisch von seiner Verschlussposition zu verdrängen, wobei das mechanische Öffnungssystem ein Gestänge (61, 62) aufweist, von dem ein erster Stangenteil (61) mit dem Blasebalg (20) einstückig ausgebildet ist und bei Betätigung der Vorrichtung in der Luftkammer (22) gleitet, und ein zweiter Stangenteil (62) bei Betätigung der Vorrichtung von dem ersten Stangenteil (61) geschoben wird, wobei das Gestänge (61, 62) am Ende des Betätigungshubs mit dem Verschlusselement (50) zusammenwirkt, um dieses mechanisch aus seiner Verschlussposition zu drängen,
wobei der Kolben (21) des Blasebalgs (20) in der Ruheposition nicht dichtend mit der Luftkammer (22) zusammenwirkt, so dass die Luftkammer (22) in der Ruheposition mit der Atmosphäre verbunden ist, wobei der Kolben (21) von dem ersten Stangenteil (61) getrennt ist und gleichzeitig in Bezug auf die Luftkammer (22) und eine zylindrische Oberfläche (614) in einem Stück mit dem ersten Stangenteil (61) gleitet, wobei der Kolben (21) eine interne Lippe (215) aufweist, die bei Betätigung der Vorrichtung dichtend auf der zylindrischen Oberfläche (614) gleitet und in der Ruheposition nicht dichtend mit auf der zylindrischen Oberfläche (614) ausgebildeten Rillen (615) zusammenwirkt, um die Luftkammer (22) in der Ruheposition der freien Luft auszusetzen, wobei der Kolben (21) in allen Positionen dichtend mit dem zylindrischen Körper (222) zusammenwirkt und nur in der Ruheposition mit der zylindrischen Oberfläche (614) nicht dichtend zusammenwirkt.

2. Vorrichtung nach Anspruch 1, wobei der Behälter (30) lösbar auf dem Blasebalg (20) montiert ist, so dass nach der Betätigung der Vorrichtung der leere Behälter von dem Blasebalg zurückgezogen und durch einen neuen, vollen Behälter ersetzt werden kann, wobei der Blasebalg (20) dazu geeignet ist, in die Ruheposition zurückzukehren, um eine neue Betätigung mit dem neuen, vollen Behälter zu erlauben.

## Claims

1. A dispenser device for dispensing a fluid or powder composition, the dispenser device including: a dispenser outlet (10); an air expeller (20) for generating a flow of air while the device is being actuated, said air expeller (20) including a piston (21) that slides in an air chamber (22) between a rest position and a dispensing position, said air chamber (22) including a cylindrical body (222) in which said piston (21) slides in airtight manner; and at least one reservoir (30) that contains a single dose of composition, said reservoir (30) including an air inlet (31) that is connected to said air expeller (20), and a composition outlet (32) that is connected to said dispenser outlet (10), said air inlet (31) including a composition retainer member (40) for retaining the composition in the reservoir (30) until the composition is dispensed, and said composition outlet (32) being closed by a closure element (50) that is force fitted in the composition outlet (32) of the reservoir (30); said device further including a mechanical opening system (61, 62) that co-operates with said closure element (50) so as to expel it mechanically from its closed position while the device is being actuated, said mechanical opening system comprising a rod assembly (61, 62), a first rod portion (61) being part of said air expeller (20) and sliding in said air chamber (22) during actuation of the device, and a second rod portion (62) pushed by said first rod portion (61) during actuation of the device, said rod assembly (61, 62) cooperating at the end of the actuation stroke with said closure element (50) to expel it mechanically from its closed portion, said piston (21) of said air expeller (20), when in its rest position, co-operating in non-airtight manner with said air chamber (22), in such a manner that said air chamber (22) is in communication with the atmosphere in the rest position, said piston (21) including an inner lip (215) that slides in airtight manner on said cylindrical surface (614) during actuation of the device, and that co-operates in non-airtight manner with fluting (615) formed on said cylindrical surface (614) in the rest position to put the air chamber (22) in communication with the atmosphere in the rest position, said piston 21) co-operating in airtight manner with said cylindrical body (222) in any positions, and co-operating in non-airtight manner with said cylindrical surface (614) only in the rest position.

2. A device according to claim 1, wherein said reservoir (30) is mounted in removable manner on said air expeller (20), such that after the device has been actuated, the empty reservoir may be removed from said air expeller and replaced by a new full reservoir, said air expeller (20) being suitable for returning into its rest position so as to enable said new full reservoir to be actuated.
